# EUROPEAN PATENT APPLICATION

(11) **EP 4 649 946 A1**
(43) Date of publication of application: **19.11.2025**
(21) Application number: 25176840.4
(22) Date of filing: 15.05.2025
(51) Int. Cl.: A61K 31/138, A61K 31/353, A61K 31/404, A61K 31/4184, A61K 45/06, A61P 9/04, A61P 9/10, A61P 9/12

(54) **COMPOSITIONS FOR TREATING ARTERIAL HYPERTENSION**

(30) Priority: 16.05.2024 IT 202400011176
(71) Applicant: Alba Research S.r.l., 71016 San Severo (FG) (IT)
(72) Inventor: RICCIONI, Graziano, 71016 San Severo (FG) (IT)
(74) Representative: Primiceri, Maria Vittoria

(57) **Abstract**

The present invention concerns a composition for the treatment of hypertensive patients, with chronic ischemic heart disease and heart failure. The invention provides a pharmaceutical composition comprising or consisting of an aliquot of:
(a) at least one compound of the pharmacological class of β-blockers, such as bisoprolol and/or nebivolol;
(b) a selective angiotensin II receptor (AT₁) inhibitor belonging to the pharmacological class of sartans, such as candesartan.

Components (a) and (b) can be associated with a third component (c) belonging to the pharmacological class of diuretics, such as indapamide.

Components (a), (b) and optionally (c) are supplied in a single formulation.

## Description

### Field of Invention

The present invention concerns a composition for the treatment of arterial hypertension, in particular the treatment of hypertensive patients with chronic ischemic heart disease and heart failure.

### Known art

Cardiovascular diseases (CVD) represent a clinical problem of extreme relevance not only epidemiologically but also economically for all health systems due to the high prevalence and chronic nature of these diseases, whose duration can be measured in decades.

In addition, CVDs involve all levels of health care, often coexist with other chronic diseases, exert a negative impact on people's quality of life, and, although effective pharmacological treatments are available, these are often not applied in clinical practice, inducing high direct and indirect costs.

The pharmacological interventions implemented must use drugs that have scientific evidence of efficacy and safety and that are targeted at patients who can benefit from them as best as possible. The clinical benefits of appropriate pharmacological treatment of cardiovascular risk factors (FRCVs) have been widely demonstrated in Randomized Controlled Clinical Trials (RCCTs), while the potential economic return has often been overlooked in the evaluation of drug use in clinical practice. The prerequisite for obtaining good clinical results from the use of drugs in CVDs is that the patient is adherent to the prescribed therapy in terms of dosage, number of drugs and duration (often *sine die*) of treatment. When drugs are used in a suboptimal way, symptoms and/or clinical conditions can worsen causing a decrease in the health status of patients and an increase in the consumption of health resources (access to medical visits, execution of tests and hospitalization).

Arterial hypertension is one of the main causes of cardiovascular morbidity and mortality in humans and it is well known that treatments that reduce blood pressure have clearly demonstrated an absolute benefit in terms of reduced mortality and morbidity.

However, despite the presence of several pharmacological classes available to reduce blood pressure, many patients continue to have poor control of blood pressure values, as found in numerous large-scale population studies. Factors that contribute to poor blood pressure control comprise:
1. **Poor patient adherence to drug therapy** associated with negative consequences on the population and the rate of which is even lower in the context of primary prevention or in chronic conditions that lead to very protracted or perennial treatments such as arterial hypertension or dyslipidaemia. Poor adherence to drug therapy is the main reason for the unsatisfactory therapeutic results obtained in several patients.
   The factors underlying poor adherence concern the **patient** (lack of social support, hesitations about drugs and their adverse effects, cultural and psychological/cognitive limitations, lack of knowledge of one's pathology and the presence of multiple comorbidities), the **prescriber** (poor communication with patients and complexity of treatment plans) and the **health** system (availability of drugs and reimbursement systems, price of the drug and access to follow-up by the patient, packaging and presentation of the different drugs and pills that change frequently).
2. **Complex guidelines** that recommend, even in the initial phase of treatment, the intake of multiple drugs for the management of the disease. Scientific studies have clearly demonstrated a plurality of etiopathogenetic mechanisms underlying the development and maintenance of hypertensive disease. For this reason, the use of active ingredients that interact with the different etiopathogenetic mechanisms allows both to reduce the different amounts of drugs (lower dosage), with a reduction in side effects (linked in most cases to an excessive dosage), and to have greater effectiveness in terms of reduction of blood pressure values and therefore of complications deriving from poor blood pressure control. The use of tablets/capsules containing multiple active ingredients capable of interacting with different mechanisms is certainly an important aspect that improves adherence to drug therapy.
3. **Therapeutic inertia** linked to the attitude of the doctor who, although aware of not achieving the objectives of the treatment (prescribing, for instance, a monotherapy at a high dosage), does not adopt a series of interventions capable of solving the problem. This aspect can be improved through the use of tablets/capsules containing different pharmacological classes with reduced dosages of the active ingredients, more effective in their effect by interacting with the different pathogenetic mechanisms, better tolerability of drugs and consequent achievement of therapeutic objectives.

In the treatment of arterial hypertension, there are several pharmacological classes in use, comprising β-blockers, ACE inhibitors, sartans, α-lytics and diuretics. In particular Nebivolol, Bisoprolol, Candesartan and Indapamide are marketed by various pharmaceutical companies both in original *branded* formulations and in equivalent/generic *unbranded formulations;* specifically, the branded formulations are:

| | **Trade name** | **Company** |
|---|---|---|
| **NEBIVOLOL** | Lobivon | Menarini |
| | Nebilox | Malesci |
| **BISOPROLOL** | Congescor | Merck |
| | Sequacor | Dompè |
| **CANDESARTAN** | Blopress | Takeda |
| | Ratacand | Astrazeneca |
| **INDAPAMIDE** | Natrilix | Servier |

There are also countless companies that market these *unbranded assets* under the most different names.

These active ingredients are largely marketed in individual dosage units for each active ingredient and, to the knowledge of the inventor, are not present in a single tablet, pill or tablet at the same time. They are also on the market with multiple dosages.

For this reason, there is a need to create new pharmacological treatment schemes aimed at attacking the various pathogenetic elements that determine both the onset and the chronicity of the diseases in question (arterial hypertension, heart failure and chronic ischemic heart disease).

### Summary of the Invention

A combination of active ingredients **is now proposed** that solves the problems highlighted above. Therefore, one of the purposes of the present invention is to provide a pharmaceutical composition comprising or consisting of an amount of:
(a) at least one compound of the pharmacological class of β-blockers, such as **bisoprolol** and **nebivolol;**
(b) a selective angiotensin II receptor (AT1) inhibitor belonging to the pharmacological class of sartans, such as **candesartan.**

Components (a) and (b) can be associated with a third component (c) belonging to the pharmacological class of diuretics, such as **indapamide.**

In some embodiments, (a) and (b) are provided in a single formulation with respective dosages.

In some embodiments, components (a), (b) and (c) are supplied in a single formulation with their respective dosages together with one or more pharmacologically acceptable components, among those known to the expert in the field.

In some embodiments, the pharmaceutical composition of the invention is suitable for oral administration.

All the above active ingredients have individually demonstrated pharmacological efficacy in numerous randomized controlled clinical trials (RCCTs) with a large number of patients treated.

In each dosing unit the dose of component (a) ranges from about 1.25 mg to about 5 mg.

In each dosing unit the dose of component (b) ranges from about 8 mg to about 32 mg.

In each dosing unit the dose of component (c) ranges from about 1.25 mg to about 2.5 mg.

In some embodiments, the pharmaceutical composition is essentially devoid of component (c).

In some embodiments, component (c) is a thiazide-like diuretic, e.g. **indapamide,** or its pharmaceutically acceptable salt or hydrate, capable of inhibiting sodium reabsorption at the distal tubular level of the nephron, facilitating its urinary secretion and stimulating diuresis without altering, unlike other diuretics, the glycaemic profile.

In some embodiments, the beta-blocker (b) is **bisoprolol** and **nebivolol,** or their pharmaceutically acceptable salt or hydrate, used both in the treatment of arterial hypertension also in combination with diuretics and in stable chronic heart failure, with both preserved and reduced left ventricular systolic function. Both these principles have a β₁-selective antagonistic action, thanks to which they can also be administered in patients with chronic obstructive pulmonary disease (COPD) and chronic peripheral arterial disease (CPAS).

In some embodiments, the angiotensin II (AT1) receptor blocker (c) is **candesartan,** or its pharmaceutically acceptable salt or hydrate, used in the treatment of high blood pressure, heart failure, and chronic ischemic heart disease. This active ingredient acts by preventing the action of angiotensin II with its receptor (AT1); this aspect is important compared to ACE inhibitors which, by blocking simple angiotensin, cause the frequent side effect of "dry cough".

In the present invention a pharmaceutical composition is also given comprising or consisting of:
a) Bisoprolol and/or Nebivolol in amounts between 1.25 and 5 mg
b) Candesartan in amounts between 8 and 32 mg

In addition, the present invention provides a pharmaceutical composition comprising:
a) Bisoprolol and/or Nebivolol in amounts between 1.25 and 5 mg
b) Candesartan in amounts between 8 and 32 mg
c) Indapamide in amounts between 1.25 and 5 mg

In addition, the present invention provides a pharmaceutical composition comprising or consisting of the following dosing units taken independently of each other:
a) Bisoprolol and/or Nebivolol in amount 1.25; 2,5; 5 mg
b) Candesartan in amount 8; 16; 32 mg

In addition, the present invention provides a pharmaceutical composition comprising or consisting of the following dosing units taken independently of each other:
a) Bisoprolol and/or Nebivolol in amount 1.25; 2,5; 5 mg
b) Candesartan in amount 8; 16; 32 mg
c) Indapamide in amount 1.25; 2,5; 5 mg

In the present invention a method is also provided for the polypharmacological treatment of hypertension, heart failure and chronic ischemic heart disease in a subject who needs the three active ingredients in dosages such as to promote an adequate titration both in reduction and increase of the dosage, necessary in the treatment of the diseases listed above.

These and other purposes and advantages of the invention will be evident from the following detailed description of the invention.

### Detailed description of the invention

In this document the terms **"treatment" or "improvement"** are used interchangeably. These terms refer to an approach aimed at achieving beneficial or desired outcomes comprising, but not limited to, therapeutic benefit and/or prophylactic benefit.

**"Therapeutic benefit"** refers to the eradication or improvement of the disorder to be treated. In addition, a therapeutic benefit is obtained by eradicating or improving one or more physiological symptoms associated with the underlying disorder in such a way that an improvement is observed in the patient, despite the patient still being affected by the underlying disorder.

For **"prophylactic benefit",** the compositions may be given to a patient at risk of developing a particular disease, or to a patient who reports one or more physiological symptoms of a disease, even if a diagnosis of that disease has not been made.

In this document:
- the wording "**β blocker"** as used here, indicates the action of selective antagonism/blockade that exerts our active ingredient (bisoprolol/nebivolol) on the β₁ receptor;
- the terms **"selective inhibitor of the AT₁ receptor"** and **"sartan"** as used here indicate a selective antagonism/blockade of our active ingredient (candesartan) on the angiotensin II (AT₁) receptor;
- the wording **"diuretic"** as used here indicates the ability of our active ingredient (indapamide) to facilitate urinary secretion and stimulate the increase in diuresis;
- the wording **"single dosage unit"** as used here, indicates a fixed formulation of two/three active ingredients in different dosages;
- the term **"polypharmacological"** as used here, indicates a set of two/three active ingredients capable of interacting with the different pathogenetic mechanisms underlying the treatment of the diseases in question; more simply, the wording **"polypharmacological"** as used here indicates the use of at least two active ingredients by the same subject, in other words, it is the simultaneous or within 24 hours intake of several drugs to treat concomitant diseases;
- The term **"about"** as used here when referring to a measurable value such as an amount, time duration, and the like, is intended to encompass variations of ±20%, ±10%, ±5%, ±1%, or ±0.1% from the specified value, wherein such variations are appropriate to perform the methods described.

The present invention relates to a composition that allows the simultaneous polypharmacological treatment of hypertension, heart failure and chronic ischemic heart disease. This treatment aims to interact with the different pathogenetic mechanisms related to the onset and chronicization of the diseases themselves and, through the use of several active ingredients in the same combination (pill/capsule/tablet), allows to carry out an effective, gradual and complete treatment that complies with the indications of the guidelines in reducing cardiovascular mortality/morbidity and improving the quality of life of patients through greater adherence to the drug therapy.

In fact, the different stages of hypertensive disease, heart failure and chronic ischemic heart disease require treatment aimed at:
1. **reducing cardiovascular mortality and morbidity.** Several studies have shown that failure to reach *target* blood pressure values significantly increases the risk of cardio-cerebrovascular events;
2. **improving the quality of life of patients.** The simplification of the therapeutic scheme through the administration of two/three active ingredients in a single formulation is a primary element in improving the quality of life of patients who in a non-negligible percentage are aged > 70 years and suffering from several chronic-disabling diseases.
3. **increasing adherence to drug therapy as much as possible with the best possible tolerability.** Poor adherence to drug therapy is the main cause behind the inadequate control of blood pressure values in hypertensive patients, with heart failure and ischemic heart disease. High adherence to drug therapy is associated not only with better blood pressure control, but above all with a reduction in the risk of coronary heart disease, myocardial infarction, heart failure and stroke.

Pharmaceutical compositions for polypharmacological treatment are provided here, comprising:
(a) at least one compound of the pharmacological class of β blockers, such as bisoprolol and/or nebivolol;
(b) a selective angiotensin II receptor (AT₁) inhibitor belonging to the pharmacological class of sartans, such as candesartan.

Components (a) and (b) can be associated with a third component (c) belonging to the pharmacological class of diuretics, such as indapamide.

In some embodiments, components (a) and (b) and optionally (c) are supplied in a single formulation that results in a pharmaceutical composition suitable for oral administration.

All the above active ingredients have individually demonstrated pharmacological efficacy in numerous randomized controlled clinical trials (RCCTs) with a large number of patients treated.

The present invention makes it possible to obtain the technical effect of a combination therapy to avoid or reduce side effects while maintaining or improving the synergistic therapeutic benefits of some combinations of drugs that are currently administered in single dosage units separated from each other.

The combination of the invention has the following characteristics:
1. **multipathogenetics:** interacts with the different mechanisms responsible for the onset and progression of the diseases to be treated;
2. **modular:** intervenes and treats the different stages of the disease since the different dosages allow to adequately manage the gradual titration [both in reduction and increase] of the ingredients thanks to the different concentrations of the active ingredients. This is an important aspect because the treatment of the diseases in question involves a gradual titration of certain drugs (e.g. β-blocker and sartan in increasing dosages). All this is useful for the prescriber who implements an appropriate therapeutic protocol appropriate to the stage of the pathologies in question, promoting better adherence to therapy. In addition, many patients are quite old, and polypharmacy characterized by numerous tablets and/or capsules can lead the patient either to mistakenly take some drugs (therapeutic confusion) or even to omit taking them due to forgetfulness or poor availability of the drug. It is known, in fact, that several drugs are supplied in a dose that is "not very titratable" so the patient is forced to "break" or divide the tablet, thus losing part of its effective dose. For this reason, there is a need to have multiple combinations of dosages in the same formulation (capsule/tablet) in order to avoid complex therapeutic schemes with the intake of numerous capsules/tablets, elements underlying poor patient adherence to therapeutic protocols.
3. **tolerated:** reduces both the adverse effects of drugs present in other fixed combinations (e.g. dry cough due to ACE inhibitors) and the secondary contraindications related to a high dosage of the individual components (bronchoconstriction in patients with COPD), accentuation of claudication in patients with CPAS; alterations in atrio-ventricular conduction).

The characteristics of the pharmaceutical composition covered by the invention is represented by being a fixed combination of two/three active ingredients with different dosages that have demonstrated both their efficacy and excellent tolerability in clinical studies published in the literature.

### Dosages

Three different dosages of bisoprolol/nebivolol and candesartan alone and in combination with indapamide.

Advantages
- Simultaneous administration of two/three effective drugs capable of significantly improving both the quality of life and the patient's adherence to drug therapy. It is well known, in fact, that all the recent Guidelines on the treatment of chronic diseases indicate a new and important *end point* to be achieved in pharmacological treatment: the improvement of quality of life and adherence to drug therapy.
- Reduction of adverse effects and contraindications. Only in the treatment of arterial hypertension, at the moment, there is a fixed combination of β blocker (bisoprolol) and ace-inhibitor (perindopril/ramipril). These combinations, in addition to being indicated only in the treatment of arterial hypertension, present, in a significant percentage of cases, the incidence of a common side effect deriving from the use of the ACE inhibitor (dry cough).
- Higher long-term tolerability and reduced risk of side effects compared to treatment with the fractionated therapeutic dose.
- Treatment of three important clinical conditions (arterial hypertension / heart failure / chronic ischemic heart disease). A very important advantage of these associations is the possibility of treating various chronic-disabling diseases.
- In the treatment of arterial hypertension there are three dosages for the different degrees of hypertensive disease (mild-moderate-severe). The possibility of different dosages also takes into account the seasonality of the therapy (during the summer climate, hypotensive therapy may need to be reduced); different dosages, therefore, facilitate the maintenance of the same therapy without having to change the type of drug.
- In heart failure there is a need to titrate the different concentrations of active ingredients over time and modulate their concentration on the basis of blood pressure values and heart rate.
- In ischemic heart disease, therapy must be modulated on the basis of certain factors, comprising heart rate and ventricular function.
- Possibility of administering a fixed combination dosage based on the presence of the present comorbidities.

The composition of the active ingredients of the invention is advantageous in terms of efficacy due to the use of important active ingredients that have documented clear efficacy in studies, being able to be used in different chronic diseases and at different stages.

The absence of an important and frequent adverse effect in the treatment of arterial hypertension represented by dry cough is an undisputed advantage compared to some associations present.

Any disadvantages deriving from the use of this combination cannot be indicated at the moment simply because in their single use all the active ingredients characterizing the combination have demonstrated both excellent pharmacological efficacy and tolerability.

In conclusion, the therapeutic effect of the composition of the invention allows:
- **Treatment of three diseases** with considerable incidence and prevalence in the population (arterial hypertension - heart failure - ischemic heart disease) through the management of an important group of patients with the most effective active ingredients available.
- **Reduction of side effects** as the single and simultaneous administration of the two/three active ingredients rather than two/three drugs administered individually at higher dosages is better tolerated.
- **Reduction of costs** deriving from the use of single principles with significant savings in health expenditure.
- **Improved quality of life.**
- **Increased adherence** to drug therapy by patients.

The pharmaceutical composition of the present invention comprises:
(a) at least one compound of the pharmacological class of β blockers, such as bisoprolol and/or nebivolol;
(b) a selective angiotensin II receptor (AT₁) inhibitor belonging to the pharmacological class of sartans, such as candesartan.

Components (a) and (b) can be associated with a third component (c) belonging to the pharmacological class of diuretics, such as indapamide.

In some embodiments, (a) and (b) are given in a single formulation.

In some embodiments, (a), (b), and (c) are given in a single formulation.

Preferably the pharmaceutical composition comprises:
(a) Bisoprolol and/or Nebivolol in amounts 1.25 - 2.5 - 5 mg
(b) Candesartan in amounts 8 - 16 - 32 mg

Preferably the pharmaceutical composition comprises:
(a) Bisoprolol and/or Nebivolol in amounts 1.25 - 2.5 - 5 mg
(b) Candesartan in amounts 8 - 16 - 32 mg
(c) Indapamide in amounts 1.25 - 2.5 mg

In the present invention, a method for the polypharmacological treatment of hypertension, heart failure and chronic ischemic heart disease is also provided in a subject who needs it, comprising the administration of the pharmaceutical composition in question.

In some embodiments, the pharmaceutical composition is essentially lactose- and gluten-free.

In some embodiments, (a), (b) and optionally (c) are given in a single formulation. In some embodiments, the pharmaceutical composition is suitable for oral administration.

In some embodiments, the composition is in the form of a pill, lozenge, capsule, or tablet.

The active ingredient can be combined, for instance, with the usual non-toxic, pharmaceutically acceptable vehicles for tablets, capsules, suppositories, solutions, emulsions, suspensions and any other form suitable for use. The active substance is included in the pharmaceutical composition in an amount sufficient to produce the desired effect on the disease process or condition.

To prepare solid compositions such as tablets, the main active ingredient may be mixed with a pharmaceutical vehicle, e.g., conventional tablet ingredients such as corn starch, lactose, sucrose, sorbitol, talc, stearic acid, magnesium stearate, dicalcium phosphate, or gums, and other pharmaceutical diluents, e.g., water, to form a solid preformulation composition containing a homogeneous mixture of a disclosed compound or its non-toxic pharmaceutically acceptable salt. When these preformulation compositions are referred to as homogeneous, it is understood that the active ingredient is dispersed evenly throughout the composition so that the composition can be easily broken down into equally effective unit dosage forms such as lozenges, pills, tablets and capsules.

In solid dosage forms for oral administration (capsules, tablets, pills, comfits, powders, granules and the like), the composition in question is mixed with one or more pharmaceutically acceptable vehicles, such as sodium citrate or dicalcium phosphate, and/or any of the following:
(1) fillers, such as starches, lactose, sucrose, glucose, mannitol, and/or silicic acid;
(2) binders such as, for instance, carboxymethylcellulose, alginates, gelatine, polyvinylpyrrolidone, sucrose and/or acacia;
(3) humectants, such as glycerol;
(4) disintegrating agents, such as agar-agar, calcium carbonate, potato or tapioca starch, alginic acid, certain silicates, and sodium carbonate;
(5) solution-retardant agents, such as paraffin;
(6) absorption accelerators, such as quaternary ammonium compounds;
(7) wetting agents, such as, for instance, acetyl alcohol and glycerol monostearate;
(8) absorbents, such as kaolin and bentonite clay;
(9) lubricants, such as talc, calcium stearate, magnesium stearate, solid polyethylene glycols, sodium lauryl sulphate, and mixtures thereof; and
(10) colouring agents.

In the case of capsules, tablets and pills, the compositions may also comprise buffering agents.

Solid compositions of a similar type can also be employed as fillers in soft and hard gelatine capsules filled using excipients such as lactose or milk sugars, as well as high molecular weight polyethylene glycols and the like.

A tablet can be made by compression or molding, optionally with one or more accessory ingredients. Tablets may be prepared using a binder (e.g. gelatine or hydroxypropylmethylcellulose), lubricant, inert diluent, preservative, disintegrator (e.g. sodium starch glycolate or cross-linked sodium carboxymethyl cellulose), or a surfactant or dispersing agent. Moulded tablets can be made by moulding a mixture of the composition in question moistened with an inert liquid thinner in a suitable machine. In some embodiments, capsules are prepared by encapsulating tablets in hard gelatine capsules. Tablets and other solid dosage forms, such as comfits, capsules, pills, and granules, may optionally be etched or prepared with coatings and shells, such as enteric coatings and other coatings well known in the art of pharmaceutical formulation.

In some embodiments, treatment results in greater long-term tolerability and reduced risk of side effects than treatment with the lowest therapeutic dose for hypertension of any of the angiotensin II receptor blocker (AT₁), diuretic, and β-blocker in the pharmaceutical composition.

In some embodiments, treatment results in greater long-term tolerability and reduced risk of side effects compared to treatment with the lowest therapeutic dose for hypertension of angiotensin II receptor blocker (AT₁) in the pharmaceutical composition.

In some embodiments, treatment results in greater long-term tolerability and a reduced risk of side effects compared to treatment with the full therapeutic dose for hypertension, lower than the diuretic in the pharmaceutical composition.

The compositions described here in some embodiments provide beneficial therapeutic effects, which comprise, but are not limited to, significant reduction in blood pressure, increased long-term tolerability, and reduced risk of side effects.

The following examples are provided to illustrate the invention and are not to be considered limiting its scope.

### EXAMPLES

### Example 1: Composition of the formulation 1

Three compositions have been created containing the following combinations of active agents:
Bisoprolol/Candesartan - 1.25 mg/8 mg
Bisoprolol/Candesartan - 2.5 mg/16 mg
Bisoprolol/Candesartan - 5 mg/32 mg

### Example 2: Composition of the formulation 2

Three compositions have been created containing the following combinations of active agents:
Bisoprolol/Candesartan/Indapamide 1.25 mg/8 mg/1.25 mg
Bisoprolol/Candesartan/Indapamide 2.5 mg/16 mg/1.25 mg
Bisoprolol/Candesartan/Indapamide 5 mg/32 mg/2.5 mg

### Example 3: Composition of the formulation 3

Three compositions have been created containing the following combinations of active agents:
Nebivolol/Candesartan - 1.25 mg/8 mg
Nebivolol/Candesartan - 2.5 mg/16 mg
Nebivolol/Candesartan - 5 mg/32 mg

### Example 4: Composition of the formulation 4

Three compositions have been created containing the following combinations of active agents:
Nebivolol/Candesartan/Indapamide 1.25 mg/8 mg/1.25 mg
Nebivolol/Candesartan/Indapamide 2.5 mg/16 mg/1.25 mg
Nebivolol/Candesartan/Indapamide 5 mg/32 mg/2.5 mg

### Clinical Study (ongoing)

### Title: Efficacy, quality of life and bioequivalence of a fixed combination of bisoprolol/nebivolol-candesartan-indapamide in the treatment of arterial hypertension.

**Introduction.** Treatment of high blood pressure often comprises combining two or more drugs with a different mechanism of action in order to achieve adequate blood pressure control. However, despite the presence of several drug classes available, many patients continue to have poor control of blood pressure values, as found in numerous large-scale population studies. Factors contributing to poor blood pressure control comprise poor patient adherence to drug therapy. Recent Guidelines recommend, even in the initial phase of treatment, the use of a fixed combination containing different pharmacological classes with reduced dosages of active ingredients, more effective in their effect by interacting with the different pathogenetic mechanisms, better tolerability of drugs and consequent achievement of therapeutic objectives.

**Aim of the study.** To evaluate the efficacy, tolerability, quality of life, and bioequivalence of a fixed combination of principles in the treatment of arterial hypertension, also in patients with chronic ischemic heart disease and heart failure.

**Patients and methods.** Randomized, open-label trial on 60 patients (18-70 years) with arterial hypertension capable of evaluating the clinical efficacy, tolerability, quality of life and bioequivalence of three different fixed combinations of bisoprolol-nebivolol/candesartan/indapamide compared to patients treated with single active substances after 4 weeks.

### Fixed combinations under trial

### Group 1

Bisoprolol/Candesartan - 1.25 mg/8 mg
Bisoprolol/Candesartan - 2.5 mg/16 mg
Bisoprolol/Candesartan - 5 mg/32 mg

### Group 2

Bisoprolol /Candesartan/Indapamide 1.25 mg/8 mg/1.25 mg
Bisoprolol /Candesartan/Indapamide 2.5 mg/16 mg/1.25 mg
Bisoprolol /Candesartan/Indapamide 5 mg/32 mg/2.5 mg

### Group 3

Nebivolol/Candesartan - 1.25 mg/8 mg
Nebivolol /Candesartan - 2.5 mg/16 mg
Nebivolol /Candesartan - 5 mg/32 mg

### Group 4

Nebivolol /Candesartan/Indapamide 1.25 mg/8 mg/1.25 mg
Nebivolol /Candesartan/Indapamide 2.5 mg/16 mg/1.25 mg
Nebivolol /Candesartan/Indapamide 5 mg/32 mg/2.5 mg

### REFERENCES

- 2018 ESC/ESH Guidelines for the management of arterial hypertension. The task force for the management of arterial hypertension of the European Society of Cardiology and the European Society of Hypertension. J of Hypert 2018; 36: 1953-2041.-2021 ESC Guidelines for the diagnosis and treatment of acute and chronic heart failure. Developed by the Task Force for the diagnosis and treatment of acute and chronic heart failure of the European Society of Cardiology (ESC). European Heart Journal 2021;42: 3599-3726.
- 2019 ESC Guidelines for the diagnosis and management of chronic coronary syndromes: The Task Force for the diagnosis and management of chronic coronary syndromes of the European Society of Cardiology (ESC). European Heart Journal 2020; 3: 407-477.
- 2023 Focus updated of the 2021 ESC guidelines for the diagnosis and treatment of acute and chronic heart failure. European Heart Journal 2023; 3:3627-3639.

## Claims

1. A pharmaceutical composition comprising an amount of:
(a) at least one compound of the pharmacological class of β-blockers;
(b) a selective angiotensin II receptor (AT₁) inhibitor belonging to the pharmacological class of sartans;
in combination with at least one pharmaceutically acceptable agent, said composition being formulated in a single dosage unit.

2. The pharmaceutical composition according to the above claim which further comprises a component (c) belonging to the pharmacological class of diuretics.

3. The pharmaceutical composition according to claims 1 or 2 wherein the β-blocker is chosen between Bisoprolol and Nebivolol and mixtures thereof; sartan is candesartan and diuretic is indapamide.

4. The pharmaceutical composition according to anyone of claims 1 to 3 wherein component (a) is present in amounts between 1.25 and 5 mg and component (b) is present in amounts between 8 and 32 mg.

5. The pharmaceutical composition according to anyone of claims 2 to 4 wherein component (a) is present in amounts between 1.25 and 5 mg, component (b) is present in amounts between 8 and 32 mg, and component (c) is present in amounts between 1.25 and 2.5 mg.

6. The pharmaceutical composition according to anyone of claims 1-5 wherein the component (a) is present in individual amounts of 1.25; 2.5; 5 mg and the component (b) is present in individual amounts of 8; 16; 32 mg.

7. The pharmaceutical composition according to anyone of claims 2-6 wherein the component (a) is present in amounts taken individually from each other 1.25; 2.5; 5 mg, the component (b) is present in amounts taken individually from each other 8; 16; 32 mg and the component (c) is present in amounts taken individually from each other 1.25; 2.5; 5 mg.

8. The pharmaceutical composition according to anyone of claims 1-5 which is chosen from one of the following formulations:
**Formulation 1**
Bisoprolol/Candesartan - 1.25 mg/8 mg
Bisoprolol/Candesartan - 2.5 mg/16 mg
Bisoprolol/Candesartan - 5 mg/32 mg
**Formulation 2**
Bisoprolol /Candesartan/Indapamide 1.25 mg/8 mg/1.25 mg
Bisoprolol /Candesartan/Indapamide 2.5 mg/16 mg/1.25 mg
Bisoprolol /Candesartan/Indapamide 5 mg/32 mg/2.5 mg
**Formulation 3**
Nebivolol/Candesartan - 1.25 mg/8 mg
Nebivolol/Candesartan - 2.5 mg/16 mg
Nebivolol/Candesartan - 5 mg/32 mg
**Formulation 4**
Nebivolol/Candesartan/Indapamide 1.25 mg/8 mg/1.25 mg
Nebivolol/Candesartan/Indapamide 2.5 mg/16 mg/1.25 mg
Nebivolol/Candesartan/Indapamide 5 mg/32 mg/2.5 mg

9. The pharmaceutical composition according to anyone of claims 1-6 in pill, lozenge, tablet or capsule form.

10. The pharmaceutical composition according to anyone of claims 1-7 that is suitable for oral administration.

11. The pharmaceutical composition according to anyone of claims 1-8 wherein the pharmaceutical composition is essentially lactose and gluten-free.

12. The pharmaceutical composition according to anyone of claims 1-9 for use in the polypharmacological treatment of hypertension, heart failure and chronic ischemic heart disease.
